# EUROPÄISCHE PATENTSCHRIFT

(11) **EP 0 870 754 B1**
(45) Veröffentlichungstag und Bekanntmachung des Hinweises auf die Patenterteilung: **31.10.2001**
(21) Anmeldenummer: 98105722.7
(22) Anmeldetag: 30.03.1998
(51) Int. Cl.: C07C 69/017, C11B 9/00

(54) **Ethylvanillinisobutyrat**
Ethyl vanillin isobutyrate
Isobutyrate d'ethylvanilline

(30) Priorität: 10.04.1997 DE 19714826
(43) Veröffentlichungstag der Anmeldung: 14.10.1998
(73) Patentinhaber: HAARMANN & REIMER GMBH, D-37601 Holzminden (DE)
(72) Erfinder: Surburg, Horst, Dr., 37603 Holzminden (DE); Esser, Peter, Dr., 37803 Holzminden (DE); Sonnenberg, Steffen, Dr., 37603 Holzminden (DE); Landi, Arturetto, 37603 Holzminden (DE); Wörner, Peter, 37603 Holzminden (DE); Güntert, Matthias, Dr., 37603 Holzminden (DE); Scheideler, Gerhard, 37671 Höxter (DE)
(74) Vertreter: Petrovicki, Wolfgang, Dr.

(56) Entgegenhaltungen:
- US-A- 5 358 930
- US-A- 5 486 502

## Beschreibung

Die Erfindung betrifft Ethylvanillinisobutyrat I (= 3-Ethoxy-4-isobutyryloxy-benzaldehyd), ein Verfahren zu seiner Herstellung und seine Verwendung als Riech- und Aromastoff.

Es gilt in der Riechstoffindustrie als allgemein akzeptierte Tatsache, daß für die Herstellung von Parfümölen weiterhin Bedarf an neuen Riechstoffen besteht, wenn diese über ihre geruchlichen Eigenschaften hinaus zusätzliche positive Sekundäreigenschaften haben, wie z.B. höhere Stabilität, geringere Tendenz zur Bildung von Verfärbungen usw., oder in der Lage sind, in einer Parfümkomposition solche Naturprodukte zu ersetzen, die sehr teuer sind und zudem oft starken Preis- und Qualitätsschwankungen unterliegen. Auf die erfindungsgemäße Verbindung, das Ethylvanillinisobutyrat, trifft beides zu.

Vanillin (4-Hydroxy-3-methoxybenzaldehyd) und Ethylvanillin (3-Ethoxy-4-hydroxybenzaldehyd) werden in großem Maße für die Komposition von Parfümölen verwendet. Sie verleihen Parfüms eine einzigartig reiche, kräftige Süße. Beide Verbindungen haben aber den Nachteil, dass sie sich in alkoholischer Lösung am Tageslicht merklich zersetzen und dadurch zu störenden braun-violetten Verfärbungen führen.

Vanillinderivate, die entweder im Handel erhältlich sind, wie Vanillinisobutyrat, oder in der Literatur beschrieben sind, wie Ethylvanillinpropionat (US-PS 5 358 930), erreichen den Geruch und Geschmack des natürlichen Vanillins bei weitem nicht, und auch ihre Lösungen weisen - wenigstens teilweise - eine störende Zersetzungstendenz am Tageslicht auf Daher bestand ein Bedarf nach einem Produkt, das die Nachteile des Standes der Technik überwindet.

Überraschenderweise ist Ethylvanillinisobutyrat hinsichtlich seiner organoleptischen Eigenschaften den bisher bekannten Vanillin- und Ethylvanillinderivaten sowohl von der Stärke als auch von der Art des organoleptischen Eindrucks her deutlich überlegen. Es zeigt einen süßen naturhaften vanilleartigen Geruch und Geschmack, der sehr stark an den kostbaren Extrakt der echten Vanilleschote erinnert, verbunden mit einer sehr appetitlichen, buttrig-cremigen Schokoladennote. Neben dem Einsatz in Parfümölen ist die erfindungsgemäße Verbindung daher auch vorzüglich geeignet, um Lebensmittel, wie z.B. Speiseeis oder Süßspeisen, zu aromatisieren.

Im Vergleich zu den ähnlichen und bereits bekannten Vanillinderivaten wie Vanillinisobutyrat und Ethylvanillinpropionat besticht das erfindungsgemäße Ethylvanillinisobutyrat auch durch seine größere Haftfestigkeit, wodurch die zeitliche Wirkung von entsprechenden Parfümölen, wenn sie z.B. auf die Haut aufgetragen werden, deutlich verlängert wird, und durch seine größere Stärke und Ausgiebigkeit (Tab. 1), die es erlauben, Parfümöle von einer vergleichbar höheren geruchlichen Stärke zu entwickeln oder in Parfümölen vergleichbare Effekte mit erheblich geringeren Dosierungen zu erzielen.

**Tabelle 1**

| Geruchliche Ausgiebigkeit von Vanillin- und Ethylvanillinisobutyrat bzw. Ethyl- vanillinpropionat | | | |
|---|---|---|---|
| Es wird getestet, welche Konzentrationen in Diethylphthalat bei Auftragen auf einen Teststreifen aus Papier ("Riechstreifen") geruchlich noch deutlich wahrnehmbar sind: | | | |

| Verdünnung | 1 Gew.-% | 0,1 Gew.-% | 0,01 Gew.-% |
|---|---|---|---|
| Vanillinisobutyrat | + | + | --- |
| Ethylvanillinisobutyrat | + | + | + |
| Ethylvanillinpropionat | + | + | --- |

Im Vergleich zu Vanillinisobutyrat und Ethylvanillinpropionat zeigt Ethylvanillinisobutyrat auch eine wesentlich bessere Lichtstabilität (s. Tabellen 2 und 3), was sich in einer geringeren Tendenz zur Verfärbung am Licht bemerkbar macht.

**Tabelle 2**

| Ermittlung der Photostabilität von Vanillin- und Ethylvanillinisobutyrat bzw. Ethylvanillinpropionat bei UV-Licht-Bestrahlung [Angaben in % der Ausgangskonzentration] | | | | | | |
|---|---|---|---|---|---|---|
| | 0 | 2h | 4h | 8h | 24h | unbestrahlt (54h) |
| Vanillinisobutyrat | 100 | 97 | 93 | 85 | 56 | 97 |
| Ethylvanillinisobutyrat | 100 | 100 | 98 | 91 | 71 | 100 |
| Ethylvanillinpropionat | 100 | 99 | 98 | 91 | 72 | 91 |

Bedingungen:
Wellenlänge: λ >290 nm; Intensität: 40 Watt/m²;
Temperatur 40°C; Konzentration: 10 gew.-%-ig in Ethanol;
Weißglasküvetten.

Das Ethylvanillinpropionat zeigt unter diesen Bedingungen eine dem Ethylvanillinisobutyrat vergleichbare Photostabilität. Werden die Verbindungen allerdings für längere Zeit dem Tageslicht ausgesetzt, so zeigt sich hier die eindeutige Überlegenheit des Ethylvanillinisobutyrats bezüglich der Lichtstabilität.

**Tabelle 3**

| Ermittlung der Lichtstabilität von Vanillin- und Ethylvanillinisobutyrat bzw. Ethylvanillinpropionat bei Bestrahlung mit Tageslicht [Angaben in % der Ausgangskonzentration] | | | | |
|---|---|---|---|---|
| | 0 | 30d | 90d | unbestrahlt (90d) |
| Vanillinisobutyrat | 100 | 80 | 30 | 95 |
| Ethylvanillinisobutyrat | 100 | 98 | 61 | 100 |
| Ethylvanillinpropionat | 100 | 88 | 31 | 97 |

Bedingungen: 10%-ige Lösungen in Ethanol; Weißglas; diffuses Tageslicht, Raumtemperatur.

Gegenstand der Erfindung ist also 3-Ethoxy-4-isobutyryloxy-benzaldehyd.

Ethylvanillinisobutyrat I ist auf an sich bekannte Weise durch Umsetzung von Ethylvanillin II mit Isobuttersäurechlorid oder Isobuttersäureanhydrid I bequem zugänglich. 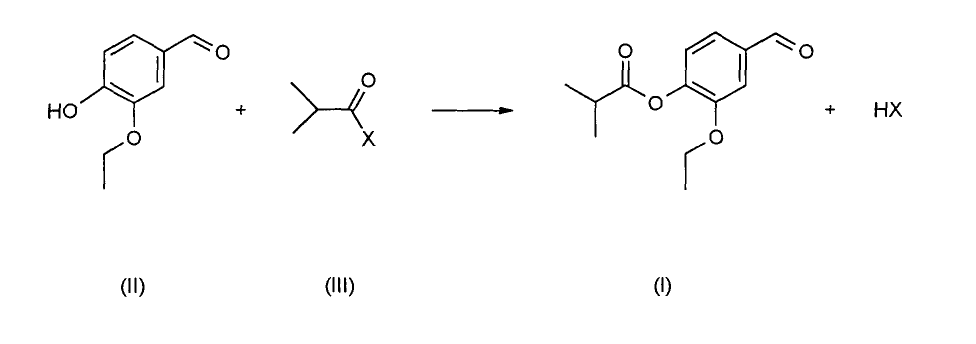 X = Cl oder -O-CO-CH(CH₃)₂.

Das Molverhältnis III/II kann in der Regel 1 bis 3, vorzugsweise 1,1 bis 2, insbesondere 1,1 bis 1,5 betragen.

Bei Verwendung von Isobuttersäurechlorid als Acylierungsmittel ist es vorteilhaft, in Gegenwart einer organischen Base, wie z.B. Pyridin, alkylsubstituierten Pyridinen, Chinolinen, tertiären Aminen wie N,N-Dimethylbenzylamin, Triethylamin, Tributylamin usw., zu arbeiten, um die freiwerdende Salzsäure abzufangen. Die zugesetzte Basenmenge beträgt dabei mindestens ein Äquivalent, bezogen auf das eingesetzte Isobuttersäurechlorid.

Wird Isobuttersäureanhydrid verwendet, kann man auf die Anwesenheit einer basischen Verbindung verzichten und die freiwerdende Isobuttersäure aus dem Reaktionsgemisch abdestillieren.

Die Reaktion kann in An- oder Abwesenheit eines unter Reaktionsbedingungen inerten organischen Lösungsmittels erfolgen. Solche Lösungsmittel umfassen beispielsweise Aromaten wie Benzol, Toluol, Xylole, lineare, verzweigte und cyclische Aliphaten wie Benzin und Cyclohexan, Ether, wie z.B. Diethylether, Ethylenglykoldimethylether, Diethylenglykoldimethylether, Tetrahydrofuran, Dioxan und Butylmethylether sowie lineare und cyclische Säureamide, wie z.B. Dimethylformamid und N-Methylpyrrolidon.

Die Reaktionstemperatur kann 0 bis 180, vorzugsweise 20 bis 140°C betragen. Das Fortschreiten der Reaktion kann durch übliche Methoden, beispielsweise mittels Dünnschichtchromatographie, verfolgt werden. Am Ende der Reaktion kann man überschüssige Mengen III abdestillieren, gegebenenfalls mit einer Base wie Natronlauge, wäßrigen Lösungen von Natriumcarbonat oder -bicarbonat neutralisieren, die organische Phase gegebenenfalls trocknen und das Reaktionsprodukt durch Destillation oder Umkristallisation reinigen.

Weiterer Gegenstand der Erfindung ist also ein Verfahren zur Herstellung von Ethylvanillinisobutyrat durch Umsetzung von Ethylvanillin mit einem reaktiven Isobuttersäurederivat.

Ethylvanillinisobutyrat eignet sich wegen seiner herausragenden organoleptischen Eigenschaften vorzüglich für den Einsatz in Parfüm- und Aroma-Kompositionen. Es läßt sich sehr gut mit anderen Riech- und Aromastoffen in verschiedenen, unterschiedlichen Mengenverhältnissen zu neuartigen Parfüm- und Aroma-Kompositionen kombinieren. In Parfümkompositionen beträgt die eingesetzte Menge 0,1 bis 20, vorzugsweise 0,5 bis 10 Gew.-%, bezogen auf die gesamte Komposition.

Derartige Parfümkompositionen können nicht nur in alkoholischer Lösung als Feinparfüms verwendet werden, sondern auch zur Parfümierung von Kosmetika, z.B. Cremes, Lotionen, Aerosolen, Toilettenseifen usw., Haushaltsprodukten wie Reinigungs- und Waschmitteln, Weichspülern, Desinfektionsmitteln und Textilbehandlungsmitteln und anderer technischer Produkte dienen, wobei die Menge der Parfüm-Komposition 0,1 bis 40, vorzugsweise 0,5 bis 20 Gew.-%, bezogen auf das ganze Produkt, beträgt.

In Aroma-Kompositionen beträgt die eingesetzte Menge der erfindungsgemäßen Verbindung 0,1 bis 50, vorzugsweise 2 bis 10 Gew.-%, bezogen auf die gesamte Komposition. Derartige Aroma-Kompositionen können im gesamten Nahrungs- und Genußmittelbereich verwendet werden. Insbesondere sind sie geeignet für die Aromatisierung von Fettmassen, Backwaren, Joghurt, Speiseeis, Süßwaren, alkoholischen und nicht- alkoholischen Getränken, Tabak und Stoffen, die für die Herstellung von Tabakerzeugnissen verwendet werden. Die Dosierung derartiger Aroma-Kompositionen beträgt 0,0005 bis 0,05, vorzugsweise 0,001 bis 0,01 Gew.-%, bezogen auf das fertige Lebens- oder Genußmittel. Im Tabak beträgt die Dosierung der erfindungsgemäßen Verbindung 0,005 bis 0,5, vorzugsweise 0,01 bis 0,1 Gew.-%, bezogen auf Tabak.

Weiterer Gegenstand der Erfindung ist also die Verwendung von Ethylvanillinisobutyrat als Riech- und Aromastoff.

Die Prozentangaben der nachfolgenden Beispiele beziehen sich jeweils auf das Gewicht; Teile sind Gewichtsteile.

### Beispiel 1: Herstellung von Ethylvanillinisobutyrat

### a) durch Umsetzung von Ethylvanillin mit Isobuttersäurechlorid:

166 g (1 Mol) Ethylvanillin und 160 g (1,33 Mol) N,N-Dimethylbenzylamin werden in 800 ml tert.- Butylmethylether unter Feuchtigkeitsausschluss in einer Rührapparatur vorgelegt. 128 g (1,2 Mol) Isobuttersäurechlorid werden während 1,5 h zudosiert, wobei die Reaktionstemperatur auf ca. 38°C ansteigt. Das Reaktionsgemisch wird bei Raumtemperatur über Nacht nachgerührt und dann auf ca. 1 l Wasser gegeben. Die organische Phase wird abgetrennt, mit Salzsäure, 5-%-iger Natronlauge und Wasser gewaschen, getrocknet und eingeengt. Als Rückstand verbleiben 238 g rohes Ethylvanillinisobutyrat, das aus 240 ml Isopropanol umkristallisiert wird. Ausbeute 215 g Ethylvanillinisobutyrat; Schmp. 57°C.
MS: M⁺= 236 (9); m/e = 166 (90), 138 (50), 137 (61), 109 (13), 81 (20), 71 (36), 51 (15), 43 (100), 41 (24), 27 (25).
¹H-NMR (200 MHz, CDCl₃): 1.34 (d, J = 7.0 Hz, 6H), 1.41 (t, J = 7.0 Hz, 3H), 2.86 (sep, J = 7.0 Hz, 1H), 4.12 (q, J = 7.0 Hz, 2H), 7.1 - 8.0 (m, 3H), 9,94 (s, 1H) δ ppm.
¹³C-NMR (90 MHz, CDCl₃): 15, 19, 34, 64, 111, 123, 125, 135, 145, 151, 175, 191 δ ppm.

### b) durch Umsetzung von Ethylvanillin mit Isobuttersäureanhydrid:

In einem 500 ml Dreihalskolben mit 30 cm Füllkörperkolonne legt man 166 g Ethylvanillin vor, schmilzt auf und dosiert bei einer Temperatur von maximal 100°C 189,6 g Isobuttersäureanhydrid zu. Die Reaktion ist nur leicht exotherm. Nach erfolgter Dosierung kann zunächst bei 120°C Sumpftemperatur und 150 mbar die iso-Buttersäure und zuletzt bei bis zu 130°C und 20 mbar iso-Buttersäure und überschüssiges Anhydrid abdestilliert werden. Man läßt auf 90°C abkühlen, dosiert 500 ml iso-Propanol und kristallisiert unter weiterem Rühren und Abkühlen auf 7°C aus. Die Kristalle werden abgesaugt, mit 2 mal 60 ml kaltem iso-Propanol ausgewaschen und getrocknet. Man erhält 182 g Ethylvanillinisobutyrat. Aus der Mutterlauge werden durch nochmalige Kristallisation weitere 22,8 g Ethylvanillinisobutyrat erhalten.

### Beispiel 2: Herstellung von Parfüms unter Verwendung von Ethylvanillinisobutyrat

### a) Herstellung eines künstlichen Vanille-Extrakts für den Einsatz in Parfümölen:

Es werden vermischt:

| | |
|---|---|
| Furaneol 0,15% in Isopropylmyristat | 7 g |
| Damascon beta 1% in Benzylalkohol | 0,6 g |
| Damascon alpha 1% in Benzylalkohol | 0,4 g |
| Guajakol 10% in Isopropylmyristat | 3 g |
| Perubalsamöl | 3 g |
| Tolu Resin 50% in Benzylbenzoat | 15 g |
| Benzoe Resin 50% in Benzylbenzoat | 7 g |
| Baummoos-Absolue 50% in Triethylcitrat | 40 g |
| Vanillin | 440 g |
| Benzylalkohol | 984 g |

Der Ersatz von 100 g des eingesetzten Benzylalkohols durch die gleiche Menge Ethylvanillinisobutyrat verleiht dieser Kompositon eine feine fruchtig-schokoladige Note, so dass der Geruchseindruck eines natürlichen Vanille-Extrakts entsteht.

### b) Herstellung eines Parfüms mit orientalischer Note:

Es werden vermischt:

| | |
|---|---|
| Vertocitral H&R | 2 g |
| Isoananat H&R | 5 g |
| Bergamotteöl dest. farbl. | 100 g |
| Mandarinenöl | 50 g |
| γ-Octalacton | 5 g |
| Profarnesol H&R | 10 g |
| Linalool | 150 g |
| Phenoxanol® IFF | 50 g |
| Rosaphen H&R | 100 g |
| Hedion® Firmenich | 200 g |
| cis-3-Hexenylsalicylat, | 13 g |
| Cumarin | 10 g |
| Hexahydroiraldein H&R | 20 g |
| Boisanol H&R | 100 g |
| Sandolen H&R | 30 g |
| Indol, 10 %ig in Dipropylenglykol | 5 g |

Zum Vergleich werden der Mischung entweder 10 g Vanillin in 140 g Dipropylenglykol oder 50 g Ethylvanillinisobutyrat in 100 g Dipropylenglykol zugesetzt. Die Komposition mit Ethylvanillinisobutyrat riecht feiner, etwas fruchtig schokoladiger, wird durch die Vanille-Extrakt-Note reicher und wertvoller und bekommt insgesamt ein elegantes Volumen.

### Beispiel 3: Herstellung einer z.B. in Speiseeis oder Süßwaren einsetzbaren Aroma-Mischung mit dem Charakter von Vanille-Extrakt unter Verwendung von Ethylvanillinisobutyrat.

Es werden vermischt:

| | |
|---|---|
| Dihydrocumarin | 1 |
| Decalacton, delta- | 1 |
| Diacetyl, 10-%ig in Propylenglykol | 1 |
| Guajakol | 1 |
| Heliotropin | 10 |
| Ethylvanillin | 50 |
| Vanillin | 200 |
| Propylenglykol | 736 |
| | 1000 |

Durch Zusatz von 20 bis 100 Teilen Ethylvanillinisobutyrat, bzw. durch Austausch des Ethylvanillins gegen Ethylvanillinisobutyrat wird das Geschmacksbild des Vanillearomas voller, süßer und harmonischer und das ganze Aroma bekommt eine sehr viel natürlichere Vanillenote, die in Richtung von echtem Vanilleextrakt geht.

## Patentansprüche

1. 3 -Ethoxy-4-isobutyryloxy-benzaldehyd.

2. Verfahren zur Herstellung von 3-Ethoxy-4-isobutyryloxy-benzaldehyd durch Umsetzung von 3-Ethoxy-4-hydroxy-benzaldehyd mit einem reaktiven Isobuttersäurederivat.

3. Verwendung von 3-Ethoxy-4-isobutyryloxy-benzaldehyd als Riech- und Aromatstoff.

4. Parfumöle mit einem Gehalt an 3-Ethoxy-4-isobutyryloxy-benzaldehyd.

5. Aromakompositionen mit einem Gehalt an 3-Ethoxy-4-isobutyryloxy-benzaldehyd.

## Claims

1. 3-Ethoxy-4-isobutyryloxybenzaldehyde.

2. Process for the preparation of 3-ethoxy-4-isobutyryloxybenzaldehyde by reacting 3-ethoxy-4-hydroxybenzaldehyde with a reactive isobutyric acid derivative.

3. Use of 3-ethoxy-4-isobutyryloxybenzaldehyde as a fragrance and flavour material.

4. Perfume oils having a 3-ethoxy-4-isobutyryloxybenzaldehyde content.

5. Flavour compositions having a 3-ethoxy-4-isobutyryloxybenzaldehyde content.

## Revendications

1. Le 3-éthoxy-4-isobutyryloxy-benzaldéhyde.

2. Procédé de préparation du 3-éthoxy-4-isobutyryloxy-benzaldéhyde par réaction du 3-éthoxy-4-hydroxy-benzaldéhyde avec un dérivé réactif de l'acide isobutyrique.

3. Utilisation du 3-éthoxy-4-isobutyryloxy-benzaldéhyde en tant que substance odorante et aromatique.

4. Parfums contenant du 3-éthoxy-4-isobutyryloxy-benzaldéhyde.

5. Compositions d'arômes contenant du 3-éthoxy-4-isobutyryloxy-benzaldéhyde.
